# EUROPEAN PATENT SPECIFICATION

(11) **EP 1 317 561 B1**
(45) Date of publication and mention of the grant of the patent: **05.08.2009**
(21) Application number: 01971567.1
(22) Date of filing: 14.09.2001
(51) Int. Cl.: C12N 15/90

(54) **MODULATION OF HOMOLOGOUS RECOMBINATION WITH SINGLE STRANDED DNA BINDING PROTEINS**
MODULATION DER HOMOLOGEN REKOMBINATION DURCH EINZELSTRANG-DNA BINDEPROTEINE
MODULATION DE LA RECOMBINAISON D'HOMOLOGUES PAR DES PROTEINES DE FIXATION DE L'ADN MONOBRIN

(30) Priority: 15.09.2000 CA 2318517
(43) Date of publication of application: 11.06.2003
(73) Proprietor: HER MAJESTY THE QUEEN IN RIGHT OF CANADA, as represented by the Minister of Agriculture and Agri-Food, Saskatoon, SK S7N 0X2 (CA)
(72) Inventor: ROZWADOWSKI, Kevin, L., Saskatoon, Saskatchewan S7N 3R1 (CA); LYDIATE, Derek, R., Saskatoon, Saskatchewan S7N 1E6 (CA)
(74) Representative: De Clercq, Ann G. Y.
(86) International application number: PCT/CA2001/001315
(87) International publication number: WO 2002/022798

(56) References cited:
- WO-A-96/22364
- WO-A-97/08331
- SHCHERBAKOVA OLGA G ET AL: "Overexpression of bacterial RecA protein stimulates homologous recombination in somatic mammalian cells." MUTATION RESEARCH., vol. 459, no. 1, 16 February 2000 (2000-02-16), pages 65-71, XP002191814 ISSN: 0027-5107
- YANEZ R J ET AL: "Gene targeting is enhanced in human cells overexpressing hRAD51." GENE THERAPY, vol. 6, no. 7, July 1999 (1999-07), pages 1282-1290, XP001057105 ISSN: 0969-7128
- COX M M ET AL: "ROLE OF SINGLE STRANDED DNA BINDING PROTEIN IN REC-A PROTEIN PROMOTED DNA STRAND EXCHANGE" JOURNAL OF BIOLOGICAL CHEMISTRY, vol. 258, no. 4, 1983, pages 2577-2585, XP001057109 ISSN: 0021-9258
- PETUKHOVA GALINA ET AL: "Single strand DNA binding and annealing activities in the yeast recombination factor Rad59." JOURNAL OF BIOLOGICAL CHEMISTRY, vol. 274, no. 48, 26 November 1999 (1999-11-26), pages 33839-33842, XP002191815 ISSN: 0021-9258
- IFTODE C ET AL: "REPLICATION PROTEIN A (RPA): THE EUKARYOTIC SSB" CRITICAL REVIEWS IN BIOCHEMISTRY AND MOLECULAR BIOLOGY, CRC PRESS, BOCA RATON, FL, US, vol. 34, no. 3, 1999, pages 141-180, XP001002021 ISSN: 1040-9238

## Description

### FIELD OF THE INVENTION

The invention is in the field of genetic manipulation of homologous recombination in meiosis and mitosis.

### BACKGROUND OF THE INVENTION

It has been suggested that recombinases may be used to stimulate mitotic homologous recombination between sister chromatids in eukaryotes, which has been proposed as a mechanism to promote gene targeting in vegetative/somatic cells. Gene targeting generally involves the directed alteration of a specific DNA sequence in its genomic locus *in vivo*. Problems have however been reported with mitotic gene targeting. It has for example been found that overexpression of RecA-homologues in mitotic cells may cause cell cycle arrest. International Patent Publication WO 97/08331 dated 6 March 1997 summarizes a range of difficulties with earlier suggestions that the *E. coli* RecA recombinase would be useful for stimulating homologous mitotic recombination (as for example had been suggested in International Patent Publications WO 93/22443, WO 94/04032 and WO 93/06221). Utilization of *E. coli* RecA in eukaryotic cells is potentially problematic because the direction of strand transfer catalysed by RecA is the opposite to the direction of strand transfer catalysed by eukaryotic RecA homologues. Nevertheless, overexpression of *E. coli* RecA has been reported to promote gene targeting approximately 10-fold in mouse cells and less than two-fold in plants. However, this latter result in plants also demonstrated a very low overall frequency of gene targeting, which would tend to cast doubt on the statistical significance of the result.

In the face of difficulties associated with the use of E. *coli* RecA in mitotic gene targeting, alternative enzymes have been used to catalyse homologous sister chromatid exchange in mitotic cells. For example, U.S. Patent Nos. 5,780,296 and 5,945,339 disclose methods to promote homologous recombination using Rec2 as an alternative recombinase to overcome problems with the use of RecA. It has been reported that overexpression of human RAD51 (homologous recombination AD51) can increase gene targeting frequency by 2-3 fold.

In applications other than gene targeting in mitotic cells, other studies have suggested that increased expression of *E. coli* RecA or RAD51 may increase the resistance of cells to radiation or other DNA damaging agents, and enhance the frequency of intrachromosomal recombination and sister-chromatid exchange.

### SUMMARY OF THE INVENTION

In one aspect of the invention, Escherichia coli single stranded DNA binding proteins (EcSSB) may be used to modulate recombination in mitosis or meiosis in a eukaryotic cell. Methods are provided for modulating homologous recombination comprising transforming a eukaryotic cell with a nucleic acid encoding a Escherichia coli single stranded DNA binding protein; and, allowing the cell to undergo mitosis or meiosis. Similarly, methods are provided for modulating homologous recombination comprising transforming a cell with an inhibitor of expression of said single stranded DNA binding protein, such as an antisense or co-suppressive nucleic acid; and, allowing the cell to undergo mitosis or meiosis.

### DETAILED DESCRIPTION OF THE INVENTION

The invention is a method of modulating homologous recombination in a eukaryotic cell comprising the use of Escherichia coli single-stranded DNA binding protein (EcSSB) or homologues thereof, wherein said homologue shares a protein sequence identity to EcSSB of at least 50%, 70%, 75%, 90% or 95%.

In a further embodiment of the invention said method further comprises transforming a eukaryotic cell with a nucleic acid encoding said single stranded DNA binding protein; and, allowing the cell to undergo mitosis or meiosis under conditions wherein the single stranded DNA binding protein is expressed at a level sufficient to modulate homologous recombination.

In a further embodiment of the invention said method further comprises transforming a eukaryotic cell with an antisense nucleic acid to inhibit the expression of said single stranded DNA binding protein; and, allowing the cell to undergo mitosis or meiosis under conditions wherein the antisense nucleic acid is expressed at a level sufficient to inhibit the expression of the single stranded DNA binding protein to modulate homologous recombination.

In a further embodiment of the invention the single stranded DNA binding protein of said methods comprises a nuclear localization sequence.

The invention is defined by the claims. Aspects not covered by the claims only serve to illustrate the utility of the claimed methods.

It is further described that meiotic or mitotic homologous recombination frequency may be modulated by modifying the activity or functional level of proteins involved in ssDNA binding. This may for example be achieved by 1) modifying the activity or level of endogenous ssDNA-binding proteins or 2) expression of heterologous ssDNA-binding proteins. In eukaryotes, a heterotrimeric complex known as RPA facilitates the ssDNA-binding activity. Therefore, the function of this complex may be modified by modifying the level or action of one or more of the subunits. Homologous recombination subunits typically possess DNA-binding domains. Accordingly, by increasing the expression or abundance of one or more of such subunits, a greater amount of ssDNA-binding potential may be provided in the cell. This may be utilized to increase conversion of ssDNA ends to a recombinogenic form suited to be a substrate for RecA-homologue proteins, such as DMC1 and RAD51, thereby increasing the frequency of homologous recombination. RPA complex is known to interact with RAD51 and DMC 1. Accordingly, methods of increasing the abundance of RPA may be used to assist in increasing homologous recombination frequency, putatively by recruiting RecA-homologues and increasing conversion of ssDNA into recombinogenic ends.

Alternatively to enhance homologous recombination frequency, methods of the invention may be used to modify the abundance or function of ssDNA binding proteins, such as RPA, or subunits thereof, to reduce homologous recombination frequency. For example, by increasing expression or function of RPA, or subunits thereof, ssDNA ends may be titrated by the ssDNA-binding proteins thereby making the ssDNA ends unavailable as substrate for RecA-homologues, thus decreasing the frequency of homologous recombination. Homologous recombination frequency may be decreased by reducing the expression or function of RPA or its subunits. This may be adapted to reduce the levels of RPA subunits to decrease conversion of ssDNA ends to a recombinogenic form, thereby decreasing the functional activity of RecA-homologues. Alternatively, RPA subunits may be engineered to have reduced function (such as reduced DNA-binding ability or protein-protein interaction ability). Reduced homologous recombination frequency may be achieved by the expression of such an altered form of protein adapted to have a dominant-negative effect, for example by reducing the formation of functional RPA complexes, or reducing the recruitment of RecA-homologues, such as by engineering the domain responsible for interaction with RAD51 and DMC1.

The effect of modifying ssDNA-binding protein level and function on increasing or decreasing homologous recombination frequency may vary depending upon the altered loci in the genome.

Expression of heterologous ssDNA-binding proteins in eukaryotic cells may be adapted to modulate the frequency of homologous recombination. *E. coli* ssDNA-binding protein (EcSSB) is thought to bind ssDNA as a single subunit up to a homotetramer. Eukaryotic RPA is believed to have arisen by duplication of an ancestral SSB. Accordingly, the prokaryotic and eukaryotic ssDNA-binding proteins are apparently structural and functional homologues. SsDN -binding proteins from bacteria, such as EcSSB or *Agrobacterium tumefaciens* VirE2, and other bacterial structural or functional homologues, or from prokaryotic or eukaryotic viruses, such as phi 29 gene 5 product, T4 phage gene 32 product, adenovirus, tobacco mosaic virus or other viral structural or functional homologues, can be used to modify homologous recombination frequency in eukaryotes. This may be achieved by overexpression of the heterologous ssDNA-binding protein, such as EcSSB, in eukaryotes. In one aspect, EcSSB may be expressed to bind to ssDNA ends created by MRE11 and associated proteins thereby titrating the ssDNA an making it less available for interaction with endogenous RPA or RecA-homologues such as RA 51 or DMC 1. By reducing the binding of RPA to ssDNA, the recruitment of RecA homologues to the ssDNA ends may be reduced (RPA is known to physically interact with RAD51 and DMC1). This may for example result in a reduction of conversion of ssDNA ends into recombinogenic ends, thereby reducing homologous recombination frequency. Alternatively, the expression of an abundance of SSB may be adapted to maintain ssDNA ends in a topology that is favourable for the action of RecA-homologues. This may be adapted to result in an increase in homologous recombination frequency (for example because of increased conversion of ssDNA ends into recombinogenic ends).

The effect of heterologous expression of ssDNA-binding proteins on increasing or decreasing homologous recombination frequency may vary depending upon the relevant expression locus (or loci) in the genome. The action of heterologous ssDNA-binding proteins in eukaryotes may be enhanced by inclusion of a nuclear localization sequence to promote accumulation of the protein in the nucleus.

Expression of EcSSB in eukaryotic cells may be adapted to promote the activity of EcRecA expressed in eukaryotic cells. For example, the activity of EcRecA in promoting homologous recombination and gene targeting in eukaryotic cells may be increased by coexpression of EcSSB alone or with a nuclear localization sequence attached to it.

Expression of EcSSB may be adapted to increase gene targeting frequency in eukaryotes, for example by conferring a favourable topology for homologous recombination to ssDNA gene targeting substrates delivered to the cell, or double-stranded DNA gene targeting substrates delivered to the cell and partially or entirely converted to ssDNA. Expression of EcSSB may be adapted to promote localization of gene targeting substrate to the nucleus of eukaryotic cells. In some instances, only a fraction of gene targeting substrate delivered to cells may actually reach the nucleus to interact with the target genomic locus. To increase the effective concentration of gene targeting substrate, the substrate may be coated *in vitro* with EcSSB engineered to encode a nuclear localization sequence (i.e. NLS-SSB). The substrate may then be transferred more efficiently to the nucleus resulting in a higher nuclear concentration of the substrate leading to higher frequency of gene targeting. NLS-SSB expressed in the host cell may for example be adapted to bind *in vivo* to linear or circular ssDNA or dsDNA substrates delivered to the cell, or T-DNA transferred by *Agrobacterium tumefaciens*, to promote transfer of the substrate to the nucleus to promote gene-targeting frequency. In the case of T-DNA from *Agrobacterium*, methods of coating the T-DNA with NLS-SSB instead of VirE2 (the normal protein coating T-DNA) may be used to promote gene targeting. This may for example be useful in embodiments where VirE2 would otherwise interact with host proteins that promote illegitimate recombination resulting in integration of the gene targeting substrate at random sites in the genome instead of the target genomic locus. In such embodiments, the replacement of VirE2 with NLS-SSB may be adapted so that the T-DNA is less affected be these host proteins and therefore has a greater likelihood of integrating at the correct targeted genomic locus.

EcSSB may be used to target proteins or peptides to RNA, particularly where those proteins or peptides normally have no or little ability to bind to DNA molecules *in vitro* and *in vivo*. Our data demonstrate that EcSSB can tolerate some additions and modifications to its structure and still bind to DNA. This has been illustrated by engineering EcSSB to encode foreign amino acids encoding a nuclear localization sequence (i.e. NLS-SSB) wherein this modified protein remains functional in its ability to interact with DNA (as illustrated by results showing NLS-SSB has a greater effect on modifying homologous recombination than SSB). Therefore, SSB or NLS-SSB may be engineered to comprise additional peptides or proteins, for example to target such molecules to interact with DNA molecules. This may for example be used to target proteins such as transcription factors, nucleases, recombinases, DNA-repair proteins, antigenic peptides, antibodies, cytotoxic agents, reporter proteins, and others.

An anti-sense molecule or a co-suppressive nucleic acid may be used to modulate the expression of SSB to modulate homologous recombination. In general, a co-suppressive nucleic acid is a nucleic acid that suppresses the expression of another nucleic acid by means of co-suppression. Anti-sense oligonucleotides, including anti-sense RNA molecules and anti-sense DNA molecules, generally act to block the translation of mRNA by binding to targeted mRNA and inhibiting protein translation from the bound mRNA. For example, anti-sense oligonucleotides complementary to regions of a DNA sequence encoding an enzyme involved in homologous recombination, such as DMC1, may be expressed in transformed cells (such as plant cells) during the appropriate developmental stage to down-regulate the enzyme. Alternative methods of down-regulating protein expression may include the use of ribozymes or other enzymatic RNA molecules (such as hammerhead RNA structures) that are capable of catalysing the cleavage of RNA (as for example disclosed in U.S. Patent Nos. 4,987,071 and 5,591,610). The mechanism of ribozyme action generally involves sequence specific hybridization of the ribozyme molecule to complementary target RNA, followed by endonucleolytic cleavage. Additionally, antibodies or peptides which inhibit the activity of the target protein may be introduced or expressed in cells to suppress activity of the target protein.

It is well known in the art that some modifications and changes can be made in the structure of a polypeptide without substantially altering the biological function of that peptide; to obtain a biologically equivalent polypeptide. In one aspect of the invention, EcSSB (E*.*coli SSB) proteins that modulate meiotic or mitotic recombination may differ from a portion of the corresponding native sequence by conservative amino acid substitutions. As used herein, the term "conserved amino acid substitutions" refers to the substitution of one amino acid for another at a given location in the peptide, where the substitution can be made without loss of function. In making such changes, substitutions of like amino acid residues can be made, for example, on the basis of relative similarity of side-chain substituents, for example, their size, charge, hydrophobicity, hydrophilicity, and the like, and such substitutions may be assayed for their effect on the function of the peptide by routine testing. In some embodiments, conserved amino acid substitutions may be made where an amino acid residue is substituted for another having a similar hydrophilicity value (e.g., within a value of plus or minus 2.0), where the following hydrophilicity values are assigned to amino acid residues (as detailed in United States Patent No. 4,554,101, incorporated herein by reference): Arg (+3.0); Lys (+3.0); Asp (+3.0); Glu (+3.0); Ser (+0.3); Asn (+0.2); Gln (+0.2); Gly (0); Pro (-0.5); Thr (-0.4); Ala (-0.5); His (-0.5); Cys (-1.0); Met (-1.3); Val (-1.5); Leu (-1.8); Ile (-1.8); Tyr (-2.3); Phe (-2.5); and Trp (-3.4). In alternative embodiments, conserved amino acid substitutions may be made where an amino acid residue is substituted for another having a similar hydropathic index (e.g., within a value of plus or minus 2.0). In such embodiments, each amino acid residue may be assigned a hydropathic index on the basis of its hydrophobicity and charge characteristics, as follows: Ile (+4.5); Val (+4.2); Leu (+3.8); Phe (+2.8); Cys (+2.5); Met (+1.9); Ala (+1.8); Gly (-0.4); Thr (-0.7); Ser (-0.8); Trp (-0.9); Tyr (-1.3); Pro (-1.6); His (-3.2); Glu (-3.5); Gln (-3.5); Asp (-3.5); Asn (-3.5); Lys (-3.9); and Arg (-4.5). In alternative embodiments, conserved amino acid substitutions may be made where an amino acid residue is substituted for another in the same class, where the amino acids are divided into non-polar, acidic, basic and neutral classes, as follows: non-polar: Ala, Val, Leu, Ile, Phe, Trp, Pro, Met; acidic: Asp, Glu; basic: Lys, Arg, His; neutral: Gly, Ser, Thr, Cys, Asn, Gln, Tyr.

Described are nucleic acid or amino acid sequences that are homologous to other sequences. An amino acid or nucleic acid sequence is "homologous" to another sequence if the two sequences are substantially identical and the functional activity of the sequences is conserved (for example, both sequences function as or encode a selected enzyme or promoter function; as used herein, the term "homologous" does not infer evolutionary relatedness). Nucleic acid sequences may also be homologous if they encode substantially identical amino acid sequences, even if the nucleic acid sequences are not themselves substantially identical, a circumstance that may for example arise as a result of the degeneracy of the genetic code.

Two nucleic acid or protein sequences are considered substantially identical if, when optimally aligned, they share at least about 25% sequence identity in protein domains essential for conserved function. In alternative embodiments, sequence identity may for example be at least 50%, 70%, 75%, 90%, 95% or any value from 25% to 99%. Optimal alignment of sequences for comparisons of identity may be conducted using a variety of algorithms, such as the local homology algorithm of Smith and Watermam,1981, Adv. Appl. Math 2: 482, the homology alignment algorithm of Needleman and Wunsch, 1970 J. Mol. Biol. 48:443, the search for similarity method of Pearson and Lipman, 1988, Proc. Natl. Acad Sci. USA 85: 2444, and the computerised implementations of these algorithms (such as GAP, BESTFIT, FASTA and TFASTA in the Wisconsin Genetics Software Package, Genetics Computer Group, Madison, WI, U.S.A.). Sequence alignment may also be carried out using the BLAST algorithm, described in Altschul et al., 1990, J. Mol. Biol. 215:403-10 (using the published default settings). Software for performing BLAST analysis may be available through the National Center for Biotechnology Information (through the internet at http://www.ncbi.nlm.nih.gov/). The BLAST algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold. Initial neighbourhood word hits act as seeds for initiating searches to find longer HSPs. The word hits are extended in both directions along each sequence for as far as the cumulative alignment score can be increased. Extension of the word hits in each direction is halted when the following parameters are met: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST programs may use as defaults a word length (W) of 11, the BLOSUM62 scoring matrix (Henikoff and Henikoff, 1992, Proc. Natl. Acad. Sci. USA 89: 10915-10919) alignments (B) of 50, expectation (E) of 10 (which may be changed in alternative embodiments to 1 or 0.1 or 0.01 or 0.001 or 0.0001; although E values much higher than 0.1 may not identify functionally similar sequences, it is useful to examine hits with lower significance, E values between 0.1 and 10, for short readings of similarity), M=5, N=4, for nucleic acids a comparison of both strands. For protein comparisons, BLASTP may be used with defaults as follows: G=11 (cost to open a gap); E=1 (cost to extend a gap); E=10 (expectation value, at this setting, 10 hits with scores equal to or better than the defined alignment score, S, are expected to occur by chance in a database of the same size of the one being searched; the E value can be increased or decreased to alter the stringency of the search); and W=3 (word size, default is 11 for BLASTN, 3 for other blast programs). The BLOSUM matrix assigns a probability score for each position in an alignment that is based on the frequency with which that substitution is known to occur among consensus blocks within related proteins. The BLOSUM62 (gap existence cost = 11; per residue gap cost =1; lambda ratio = 0.85) substitution matrix is used by default in BLAST 2.0. A variety of other matrices may be used as alternatives to BLOSUM62, including: PAM30 (9,1,0.87); PAM70 (10,1,0.87) BLOSUM80 (10,1,0.87); BLOSUM62 (11,1,0.82) and BLOSUM45 (14,2,0.87). One measure of the statistical similarity between two sequences using the BLAST algorithm is the smallest sum probability (P(N)), which provides an indication of the probability by which a match between two nucleotide or amino acid sequences would occur by chance. In alternative embodiments of the invention, nucleotide or amino acid sequences are considered substantially identical if the smallest sum probability in a comparison of the test sequences is less than about 1, preferably less than about 0.1, more preferably less than about 0.01, and most preferably less than about 0.001.

An alternative indication that two nucleic acid sequences are substantially identical is that the two sequences hybridize to each other under moderately stringent, or preferably stringent, conditions. Hybridization to filter-bound sequences under moderately stringent conditions may, for example, be performed in 0.5 M NaHP0₄, 7% sodium dodecyl sulfate (SDS), 1 mM EDTA at 65°C, and washing in 0.2 x SSC/0.1% SDS at 42°C (see Ausubel, et al. (eds), 1989, Current Protocols in Molecular Biology, Vol. 1, Green Publishing Associates, Inc., and John Wiley & Sons, Inc., New York, at p. 2.10.3). Alternatively, hybridization to filter-bound sequences under stringent conditions may, for example, be performed in 0.5 M NaHPO₄, 7% SDS, 1mM EDTA at 65°C, and washing in 0.1 x SSC/0.1% SDS at 68°C (see Ausubel, *et al*.(*eds*), 1989, *supra*.). Hybridization conditions may be modified in accordance with known methods depending on the sequence of interest (see Tij ssen, 1993, Laboratory Techniques in Biochemistry and Molecular Biology -- Hybridization with Nucleic Acid Probes, Part I Chapter 2 "Overview of principles of hybridization and the strategy f nucleic acid probe assays", Elsevier, New York). Generally, stringent conditions are selected to be about 5°C lower than the thermal melting point for the specific sequence at a defined ionic strength and pH.

### EXAMPLE 1

Template for amplifying SSB was genomic DNA from *E. coli* strain CC 106. Genomic DNA from the strain was isolated as follows: 1) cells were cultured to mid-log phase in TYS liquid medium at 37°C; 2) cells were pelleted by centrifugation and washed with sterile distilled water; 3) 20 ml of cell culture vas centrifuged and the cell pellet resuspended in 0.5 ml TE/Tween buffer (10 mM Tris-HCl (pH 8.0), 1 mM EDTA, 0.01% (w/v) Tween 20); 4) cells were incubated at 85°C for 20-30 min and then pelleted by microcentrifugation for 5-10 min; 5) the supernatant was collected at 50 µl of TE-RNase (RNase A 20 micrograms/ml) was added before incubation at room temperature for 30 min; 6) the supernatant was extracted with phenol and chloroform and precipitated with ethanol as per standard procedure. 7) the DNA was resuspended in 20µl of LTE (TE) diluted 1:10 with distilled water).

The SSB gene was amplified with two primer sets to create two clones of the gene with restrictions sites at the 5' end. PCR reactions were performed with 4 µl of genomic DNA, 1.0 pmol SSB-5'Bam oligo and 1.0 pmol SSB-3Pst oligo, or 1.0 pmol SSB-Sma oligo and 1.0 pmol SSB-3'Pst oligo, plus 0.2 mM dNTP's, 2.5 U Pfu (Stratagene) and Pfu buffer constituents recommended by the manufacturer in a volume of 50 µl. The PCR conditions were 5 min @ 94°C, followed by 25 cycles of 30 s @ 94°C, 30 s @ 55°C and 1.0 min @ 72°C, followed by 10 min @ 72°C and storage at 4°C or -20°C. The amplified DNA from the PCR reactions using SSB-5'Bam oligo and SSB-3'Pst oligo or SSB-5'Sma oligo and SSB-3'Pst oligo was digested with BamHI and PstI or SmaI and PstI, respectively. The corresponding plasmid cloning vector pBluescript II KS (Stratagene) was also digested with BamHI and PstI or SmaI and PstI, respectively. DNA fragments of interest corresponding to SSB (~0.55 kb) and the vector (~3 kb) were purified by agarose gel electrophoresis and recovered from the agarose as described above. The fragments were ligated together, transformed into *E. coli* and putative clones of the SSB gene identified as described above. The DNA sequence of the resultant clones, pTK27 and PTK28, were determined to confirm they encoded SSB with flanking restriction sites of BamHI and PstI or SmaI and PstI, respectively.

A SSB derivative was also created so that the resultant protein would encode a nuclear localization sequence (i.e. NLS-SSB). A synthetic oligonucleotide was created which encoded the nuclear localization sequence (NLS) corresponding to that found in simian virus 40 T-antigen. The nucleotide sequence (GGATCCAAAAAAATGGCTCCTAAGAAGAAG-AGAAAGGTTGGAGGAGGACCCGGG) encodes a BamHI site, in-frame start codon, and Smal site (underlined). A plasmid containing this cloned NLS sequence and derived from pBluescript II KS- (Stratagene) was digested with SmaI and PstI and the DNA fragment corresponding to the vector (~ 3 kb) was gel purified. pTK28 was also digested with SmaI and PstI and the fragement pTK28 was also digested with SmaI and PstI and the DNA fragment corresponding to the SSB gene (~0.55 kb) was also gel purified. The DNA fragments were recovered from agarose, ligated together, transformed into *E. coli* and putative clones of the NLS-SSB gene identified as described above. The DNA sequence of the resultant clone, pTK29, was determined to confirm it encoded NLS-SSB.

### EcSSB

To demonstrate the effect of increased ssDNA-binding protein on homologous recombination frequency, SSB and NLS-SSB were cloned and expressed in *S. cerevisiae* cells undergoing meiosis. SSB and NLS were cloned individually into pCM190 by first digesting this vector with BamHI and PstI. pTK27 (SSB) and pTK29 (NLS-SSB) were also digested with BamHI and PstI. DNA fragments of interest corresponding to SSB or NLS-SSB (~0.6 kb) and pCM190 (~8 kb) were purified by agarose gel electrophoresis and recovered from the agarose as described above. The SSB and NLS-SSB DNA fragments were ligated independently to the DNA fragment corresponding to pCM190, transformed into *E. coli* and putative clones of the genes in the expression vector were identified. The resultant clones of SSB and NLS-SSB in pCM190 were denoted pTK35 and pTK36, respectively.

### Biological Assay

To demonstrate the effect of SSB on homologous recombination frequency, plasmids containing the candidate genes were introduced into *S. cerevisiae* BR2495 to create different strains. These strains were the grown, induced to undergo meiosis and the resultant progeny scored for phenotypic markers to determine homologous recombination frequency. Comparison of the homologous recombination frequency in the various strains to control strains containing parental vector containing no test gene enabled assessment of the genetic and biological effects of the test genes.

Expression vectors containing the test genes were introduced into *S*. *cerevisiae*. Exemplified genes and the corresponding expression plasmids are outlined in TABLE 1. Cell lines carrying plasmid constructs were selected by culturing cells in minimal medium lacking uracil (i.e. SC-URA; [1]): BR2495 is homozygous for the defective *ura3-1* allele [2] and therefore cannot synthesize this essential metabolite; expression plasmids based on pCM188, pCM189 and pCM190 have a functional URA3 gene and therefore BR2495 cells possessing such plasmids will be able to synthesize uracil and be able to grow on medium lacking uracil. Cells were cultured in the presence of doxycycline (10 µg/ml for solid media; 5 µg/ml for liquid media) to suppress expression of test genes until desired growth stages.

To assay homologous recombination frequency, single colonies from each test strain were used to first inoculate 3 ml of SC-URA+DOX (i.e. SC-URA containing doxycycline at 5 µg/ml) in a 15 ml tube (Falcon) which was then incubated at 30°C with shaking (200 RPM) for ~1.5 d. Ten cultures were prepared for each test strain, including BR2495 possessing the parental expression vector without a test gene and BR2495 possessing the various expression plasmids containing the test genes. Cells from 1 ml of culture were pelleted by centrifugation at 9000 RPM for 2 min in a standard microcentrifuge (Brinkman) and resuspended in 1 ml of sterile-distilled water (SDW). The cells were used to inoculate 5 ml of SC-A pre-meiosis medium (per litre: 1.7 g yeast nitrogen free base (Difco), 5 g ammonium acetate (Sigma), 20 g potassium acetate (Sigma), 2 g amino acid drop out mix; and in some experiments doxycycline at 5 µg/ml) in a 50 ml tube (Falcon) at a 1:50 dilution. The cultures were then incubated at 30°C with shaking (225 RPM) for 2 d. Aliquots of cells from each culture were then collected to assay for mitotic homologous recombination frequency occurring during vegetative growth. Dilutions of these cells were plated on YPD medium (per litre: 10 g Bacto-yeast extract, 20 g Bacto-peptone, 20 g glucose, 20 g Bacto-agar; [1]) to determine viable cell number, and plated on minimal media lacking particular amino acids so as to examine homologous recombination at different test genomic loci in BR2495 (i.e. SC minus histidine (SC-his), leucine (SC-leu), threonine (SC-thr), or tryptophan (SC-trp) [1]). These plates were incubated at 30°C for 2-4 d and then colonies were counted. The remaining cells in each culture in pre-meiosis medium were pelleted by centrifugation at 4000 RPM for 10 min at 4°C. For cultures containing doxycyline in the pre-meiosis medium, the pellet was resuspended in 5 ml of SC-A pre-meiosis medium and incubated at room temperature for 3 hours. These cells, and those cells incubated in pre-meiosis medium without doxycycline, were then pelleted by centrifugation at 4000 RPM for 10 min at 4°C and resuspended in 4 ml SPM meiosis-induction medium (0.3% (w/v) potassium acetate, 0.02% (w/v) raffinose 5 µg/ml histidine, 25 µg/ml leucine, 5 µg/ml tryptophan, 50 µg/ml threonine, 5 µg/ml adenine). The cells were again pelleted by centrifugation at 4000 RPM for 10 min at 4°C and resuspended in 3.5 ml SPM meiosis-induction medium. The cultures were then incubated at 30°C with shaking (225 RPM) for 2 d (days) to enable cells to undergo meiosis. Dilutions of the cells were made using SDW and cells were then plated on YPD to determine viable cell number, and on minimal media lacking particular amino acids so as to examine homologous recombination at different test genomic loci in BR2495, as described above. Duplicate dilutions and plating of each culture were performed. Plates were incubated at 30°C for 2-4 d and then colonies were counted. Frequency of recombinants for each culture was determined by dividing the number of prototrophs conferred by restoration of function for a particular test locus heteroallele by the viable cell number, taking into consideration the dilution factors. Meiotic homologous recombination frequency for each culture was corrected when necessary for background recombinants resulting during vegetative growth by subtraction of the mitotic homologous recombination frequency determined prior to placing the cells in SPM meiosis-induction medium. Mean values for the 10 replicates of each test strain were determined using the corrected values. Inclusion of the values from all 10 replicates in determining the mean was evaluated by the Q- test and values from individual replicates were excluded from the final mean if the statistic indicted a significant deviation from the values of other replicates. Comparison of means of meiotic homologous recombination frequency from test genes to that from control strains was done to determine the effect of the test gene. Statistical significance of the differences between these values was confirmed by evaluation using the t-test [3].

### Results

As shown in TABLE 1, results from the exemplified embodiments demonstrate modification of homologous recombination frequency (increases and decreases) through modifying the expression and activity of SSB.

Heterologous expression of a ssDNA-binding protein, SSB, is shown to decrease or increase homologous recombination frequency depending upon the genomic locus. A dominant-negative effect results at some loci to suppress homologous recombination, as shown at the *leu2* locus where a reduction of homologous recombination frequency by ~40% was shown. In contrast, a stimulation of homologous recombination occurs at some loci, as shown by the *his4* locus where homologous recombination frequency was enhanced by 13%. In both cases, the action of SSB in eukaryotes was promoted ~10% by addition of a nuclear localization sequence to the protein (i.e. NLS-SSB). In alternative embodiments, modifying the function or expression of endogenous native ssDNA-binding proteins may also be used to modify meiotic homologous recombination frequency.

### References

1. Adams, A., et al., Methods in Yeast Genetics, Cold Spring Harbour Laboratory Press, 1987.
2. Ross-MacDonald, P. et al., "Mutation of a meiosis specific MutS homologue decreases crossing over but not mismatch correction", Cell, 79: 1069-1080, 1994.
3. Devore, J.L., Probability and Statistics, Duxbury Press, 1995.

| | | | | | | | | | | | | | | |
|---|---|---|---|---|---|---|---|---|---|---|---|---|---|---|
| Table 1 | | | | | | | | | | | | | | |

| | Expression Plasmid | | | | | *His4* | | | *Leu2* | | | *Trp1* | | |
|---|---|---|---|---|---|---|---|---|---|---|---|---|---|---|
| Gene | Experiment | Plasmid Construct | Vector | Promoter^{b} | Copy Number^{c} | Prototroph Frequency^{d} | Ratio of HR^{e} | Mean Ratio of HR | Prototroph Frequency | Ratio of HR | Mean Ratio of HR | Prototroph Frequency | Ratio of HR | Mean Ratio of HR |
| SSB | 1 | control^{a} | pCM190 | Strong | High | 6.96x10⁻³ | 1.13 | 1.13 | 1.58x10⁻⁴ | 0.61 | 0.61 | | | |
| | | pTK35 | pCM190 | Strong | High | 7.89x10⁻³ | | | 9.58x10⁻⁵ | | | | | |
| | | | | | | | | | | | | | | |
| NLS-SSB | 1 | control | pCM190 | Strong | High | 6.96x10⁻³ | 1.29 | 1.29 | 1.58x10⁻⁴ | 0.54 | 0.54 | | | |
| | | pTK36 | pCM190 | Strong | High | 9.00x10⁻³ | | | 8.61x10⁻⁵ | | | | | |

| | | | | | | | | | | | | | | |
|---|---|---|---|---|---|---|---|---|---|---|---|---|---|---|
| ^{a}Control plasmid contained no gene for expression. ^{b}Promoter strength was indicated as "weak" for plasmids containing 2 copies of *tetO* and "strong' for plasmids containing 7 copies of *tetO.* ^{c}Plasmid copy number was "low" with 1-2 copies per cell and "high" for plasmids with upto 40 copies per cell. ^{d}Prototroph frequency determined as the number of prototrophs per viable cell number. Value represents the mean from 10 independent cultures. ^{e}Meiotic homologous recombination frequency determined by dividing the prototroph frequency of the strain with the test gene with that of the control. | | | | | | | | | | | | | | |

## Claims

1. A method of modulating homologous recombination in a eukaryotic cell comprising the use of Escherichia coli single-stranded DNA binding protein (EcSSB) or homologues thereof, wherein said homologue shares a protein sequence identity to EcSSB of at least 50%, 70%, 75%, 90% or 95%.

2. The method of claim 1, comprising transforming a eukaryotic cell with a nucleic acid encoding said single stranded DNA binding protein; and, allowing the cell to undergo mitosis or meiosis under conditions wherein the single stranded DNA binding protein is expressed at a level sufficient to modulate homologous recombination.

3. The method of claim 1, comprising transforming a eukaryotic cell with an antisense nucleic acid to inhibit the expression of said single stranded DNA binding protein; and, allowing the cell to undergo mitosis or meiosis under conditions wherein the antisense nucleic acid is expressed at a level sufficient to inhibit the expression of the single stranded DNA binding protein to modulate homologous recombination.

4. The method of claims 1 or 2, wherein the single stranded DNA binding protein comprises a nuclear localization sequence.

## Patentansprüche

1. Verfahren zum Modulieren homologer Rekombination in einer eukaryotischen Zelle, das die Verwendung des DNA-Einzelstrangbindeproteins von Escherichia coli (EcSSB) oder Homologen davon umfasst, wobei das Homolog eine Proteinsequenzidentität mit EcSSB von mindestens 50 %, 70 %, 75 %, 90 % oder 95 % teilt.

2. Verfahren nach Anspruch 1, das das Umwandeln einer eukaryotischen Zelle mit einer Nukleinsäure umfasst, die das DNA-Einzelstrangbindeprotein kodiert; und, Ermöglichen, dass es bei der Zelle zu Mitose oder Meiose kommt, unter Bedingungen, bei denen das DNA-Einzelstrangbindeprotein mit einem Spiegel exprimiert wird, der ausreicht, um homologe Rekombination zu modulieren.

3. Verfahren nach Anspruch 1, das das Umwandeln einer eukaryotischen Zelle mit einer Antisense-Nukleinsäure umfasst, um die Expression eines DNA-Einzelstrangbindeproteins zu inhibieren; und, Ermöglichen, dass es bei der Zelle zu Mitose oder Meiose kommt, unter Bedingungen, bei denen die Antisense-Nukleinsäure mit einem Spiegel exprimiert wird, der ausreicht, um die Expression des DNA-Einzelstrangbindeproteins zu inhibieren, um homologe Rekombination zu modulieren.

4. Verfahren nach den Ansprüchen 1 oder 2, wobei das DNA-Einzelstrangbindeprotein eine Zellkern-Lokalisierungssequenz umfasst.

## Revendications

1. Procédé de modulation de la recombinaison homologue dans une cellule eucaryote qui comprend l'utilisation de la protéine de liaison de l'ADN monocaténaire d'Escherichia coli (EcSSB) ou d'homologues de celle-ci, ledit homologue partageant une identité de séquence protéique avec EcSSB d'au moins 50 %, 70 %, 75 %, 90 % ou 95 %.

2. Procédé selon la revendication 1, qui comprend la transformation d'une cellule eucaryote avec un acide nucléique codant pour ladite protéine de liaison de l'ADN monocaténaire ; et le fait de permettre à la cellule de subir la mitose ou la méiose dans des conditions dans lesquelles la protéine de liaison de l'ADN monocaténaire est exprimée à un niveau suffisant pour moduler la recombinaison homologue.

3. Procédé selon la revendication 1, qui comprend la transformation d'une cellule eucaryote avec un acide nucléique antisens pour inhiber l'expression de ladite protéine de liaison à l'ADN monocaténaire ; et le fait de permettre à la cellule de subir la mitose ou la méiose dans des conditions dans lesquelles l'acide nucléique antisens est exprimé à un niveau suffisant pour inhiber l'expression de la protéine de liaison de l'ADN monocaténaire pour moduler la recombinaison homologue.

4. Procédé selon les revendications 1 ou 2, dans lequel la protéine de liaison de l'ADN monocaténaire comprend une séquence de localisation nucléaire.
